# EUROPEAN PATENT APPLICATION

(11) **EP 4 640 702 A1**
(43) Date of publication of application: **29.10.2025**
(21) Application number: 23907653.2
(22) Date of filing: 18.12.2023
(51) Int. Cl.: C07K 16/18, A61K 9/00, A61P 27/02, A61K 39/00

(54) **USE OF ALPHA-2,6-SIALYLATED IMMUNOGLOBULIN IN PREVENTING OR TREATING DRY EYE SYNDROME OR INFLAMMATORY EYE DISEASES**

(30) Priority: 21.12.2022 KR 20220180960
(71) Applicant: Konkuk University Industrial Cooperation Corp, Seoul 05029 (KR)
(72) Inventor: KANG, Young-Sun, Seoul 05268 (KR); KIM, Joon Young, Seoul 05119 (KR); CHOI, Hyeongjwa, Seoul 05010 (KR); LEE, Haeun, Seoul 02084 (KR)
(74) Representative: Dehns
(86) International application number: PCT/KR2023/020869
(87) International publication number: WO 2024/136368

(57) **Abstract**

The present invention relates to the use of α-2,6-sialylated immunoglobulin derived from human blood in prevent or treating dry eye syndrome or inflammatory eye diseases. The α-2,6-sialylated immunoglobulin according to the present invention, compared to IVIG, exhibits excellent therapeutic effects for dry eye syndrome even at a low dose without side effects such as fibrosis of corneal tissue, and also for intractable inflammatory eye diseases of varying severity.

## Description

### Technical Field

The present invention relates to the use of α-2,6-sialylated immunoglobulin for the prevention or treatment of dry eye syndrome or inflammatory eye diseases, more specifically the present invention relates to the use of α-2,6-sialylated immunoglobulin derived from human blood, for the prevention or treatment of dry eye syndrome or inflammatory eye diseases by inducing DC-SIGN-mediated immune tolerance.

### Related Art

The CH2 domain of the Fc region of immunoglobulin (Ig) contains a conserved asparagine residue at position 297 (Asn297), which undergoes glycosylation in more than 30 distinct forms. Among these, α-2,6-sialylated immunoglobulin (α-2,6-SA-IG) refers to an Ig in which the terminal sialic acid is attached at the 2,6-position of the glycan structure linked to Asn297. The glycosylation profile of Ig plays a critical role in its function. Notably, fully sialylated α-2,6-SA-IG constitutes less than 5% of total serum Ig, and due to the structural diversity of over 30 covalently bound glycans in the CH2 region, its proportion is likely to be even lower.

Terminal sialylation of glycan chains, referred to as "capping," serves as a key determinant of the in vivo half-life of glycoproteins. Glycoproteins lacking terminal sialic acids are rapidly cleared from the circulation. In the case of Ig, the glycan chains attached to Asn297 are located between the two heavy chains forming a dimer. The type and structure of the glycans bound to the Fc region influence the tertiary structure of the Fc, which in turn affects its interaction with Fc receptors (such as Fcγ, FcγRII, and FcγRIII).

Given that Fc-mediated functions such as antibody-dependent cellular cytotoxicity (ADCC) and complement-dependent cytotoxicity (CDC) are key determinants of therapeutic antibody efficacy, the glycosylation pattern directly impacts antibody function. Antibodies lacking Fc glycosylation are significantly impaired in their effector functions (Nature Rev Immunol. 8:34-47).

Meanwhile, mouse SIGN-R1/human DC-SIGN are receptors that recognize α-2,6-sialylated IgG Fc, which is essential for the anti-inflammatory activity of IVIG (Proc Natl Acad Sci U S A. 2008 Dec 16; 105(50):19571-8). Furthermore, it has been reported that upon administration of IVIG, the sialylated Fc portion of IVIG binds to monocytes expressing human DC-SIGN, leading to the production of IL-33. IL-33 then activates endogenous Fcε-expressing leukocytes to induce IL-4 production, which subsequently upregulates FcγRIIb on macrophages, thereby increasing the threshold required for initiation of inflammatory responses and making inflammation less likely to occur(Nature. 2011 Jun 19; 475(7354):110-3).

IVIG is prepared by isolating the Ig fraction from human donor blood, and thus the secure procurement and stable storage of healthy blood are essential. Moreover, the cost of a single IVIG injection remains extremely high. IVIG is widely used in the treatment of various immune-related diseases and, when administered intravenously, exerts potent anti-inflammatory effects, resulting in continuously increasing demand.

The present inventors previously developed a method for isolating α-2,6-sialylated immunoglobulin (α-2,6-SA-IG) from IVIG. Upon intravenous administration of the isolated α-2,6-SA-IG(HB α-2,6-SA-IVIG), they confirmed the induction of DC-SIGN-mediated immune tolerance. In particular, in patients with preeclampsia-a condition characterized by a marked reduction in the number of DC-SIGN-expressing Hofbauer cells and decreased IL-10 expression at the time of delivery-immune imbalance was significantly alleviated. On this basis, the inventors proposed the potential utility of HB α-2,6-SA-IVIG for the prevention or treatment of preeclampsia (Korean Registered Patent No. 10-2549282). However, the therapeutic application of HB α-2,6-SA-IVIG in eye diseases has not yet been reported.

Dry eye syndrome is a disorder of the ocular surface caused by insufficient tear production, excessive tear evaporation, or an imbalance in the composition of the tear film, resulting in ocular surface damage accompanied by symptoms such as stinging, irritation, foreign body sensation, and dryness. When dry eye syndrome develops, the tear film, which normally protects and evenly coats the ocular surface, rapidly breaks up, leading to symptoms such as ocular discomfort, a gritty or sandy sensation, blurred vision, and, in severe cases, even headaches. Recently, the prevalence of dry eye syndrome has been increasing due to factors such as prolonged exposure to digital devices, complications following LASIK or LASEK surgery, and environmental pollution, thereby emphasizing the growing importance of proper treatment and management of the condition.

Inflammatory eye diseases refer to a broad spectrum of conditions characterized by inflammation affecting the eye or its surrounding tissues. These include common conditions such as allergic conjunctivitis induced by hay fever, as well as rarer but more severe diseases such as uveitis, scleritis, optic neuritis, keratitis, retinal vasculitis, and chronic vasculitis. All of these diseases pose a potential threat to vision if left untreated.

To treat dry eye syndrome, artificial tear eye drops and tear secretagogues are commonly used. However, single-use artificial tear preparations are relatively expensive and, while they may alleviate the symptoms of dry eye, they do not provide a curative effect. Multi-use formulations often contain preservatives, which can induce eye inflammation. Tear secretagogues, on the other hand, promote the secretion of mucin and aqueous components but do not suppress inflammation. Therefore, their therapeutic efficacy is limited in cases of dry eye syndrome accompanied by significant inflammation.

In addition, anti-inflammatory agents administered to treat inflammatory eye diseases are effective for short-term use but may increase the risk of infection due to immunosuppression. Their use is also associated with a higher incidence of cataracts and glaucoma, and prolonged administration may lead to systemic side effects.

Thus, although the prevalence of dry eye syndrome and inflammatory eye diseases is increasing, appropriate therapeutic options for effectively treating these conditions remain insufficient, highlighting the urgent need for the development of more effective and safer treatments.

### SUMMARY OF THE INVENTION

### TECHNICAL PROBLEM

The present inventors, as a result of extensive research efforts to develop a safe and highly effective therapeutic agent for dry eye syndrome and inflammatory eye diseases, have confirmed that HB α-2,6-SA-IVIG, which is isolated from intravenous immunoglobulin (IVIG) currently approved for therapeutic use, can exert preventive, ameliorative, and/or therapeutic effects on dry eye syndrome and inflammatory eye diseases, thereby completing the present invention.

Accordingly, an object of the present invention is to provide a novel composition for the prevention, alleviation, or treatment of dry eye syndrome and inflammatory eye diseases.

### TECHNICAL SOLUTION

To achieve the above objects, the present invention provides a pharmaceutical composition for preventing or treating dry eye syndrome or inflammatory eye disease, comprising sialylated immunoglobulin.

In the present invention, the immunoglobulin may be isolated from intravenous immunoglobulin (IVIG).

In the present invention, the sialylated immunoglobulin may include α-2,6-sialylated immunoglobulin.

In the present invention, the inflammatory eye disease may be an inflammatory eye disease of the eye, oscular surface, posterior segment of the eye or periocular region.

In the present invention, the inflammatory eye disease may include at least one selected from the group consisting of hordeolum (stye), blepharitis, lid wiper epitheliopathy, chalazion, eyelid infection, ulcerative and non-ulcerative keratitis, conjunctivitis, anterior uveitis, conjunctival ulcer, iridocyclitis, scleritis, episcleritis, optic neuritis, retinal vasculitis, chronic vasculitis, posterior uveitis, pan uveitis, meibomian gland dysfunction (MGD), and dacryoadenitis.

In the present invention, the pharmaceutical composition may be co-administered with other therapeutic agent for treating dry eye syndrome or inflammatory eye disease.

In the present invention, the other therapeutic agent for treating dry eye syndrome or inflammatory eye disease may include at least one selected from the group consisting of an anti-inflammatory agent, an immunosuppressant, an anti-glaucoma agent, artificial tears, a tear secretion stimulant and an antibiotic.

In the present invention, the pharmaceutical composition may be in the form of an ophthalmic solution or an ointment.

In the present invention, the α-2,6-sialylated immunoglobulin, as compared to conventional intravenous immunoglobulin,
(i) may enhance the expression of IL-10, an anti-inflammatory cytokine in ocular cells, by 50% or more; and/or
(ii) may not induce fibrotic side effects in the corneal stroma.

In addition, the present invention provides ophthalmic composition, comprising α-2,6-sialylated immunoglobulin.

In the present invention, the ophthalmic composition may be in the form of a solution or an ointment.

In the present invention, the ophthalmic composition may include an artificial tear solution.

In addition, the present invention provides a method for treating dry eye syndrome or inflammatory eye disease, comprising administering to subject in need thereof the pharmaceutical composition.

In the present invention, the subject may be an animal including a human or non-human animal.

In the present invention, the pharmaceutical composition may be administered simultaneously, separately, or sequentially with other therapeutic agent for treating dry eye syndrome or inflammatory eye disease.

In addition, the present invention provides a use of α-2,6-sialylated immunoglobulin or the pharmaceutical composition for preventing or treating dry eye syndrome or inflammatory eye diseases.

In addition, the present invention provides a use of α-2,6-sialylated immunoglobulin or the pharmaceutical composition in the preparation of a medicament for preventing or treating dry eye syndrome or inflammatory eye diseases.

### ADVANTAGEOUS EFFECTS

The α-2,6-sialylated immunoglobulin according to the present invention exhibits excellent therapeutic effects on dry eye syndrome even at a low dose, without causing side effects such as corneal fibrosis that may occur with IVIG. In addition, it has the advantage of demonstrating outstanding therapeutic efficacy against various severe or refractory inflammatory eye diseases.

### BRIEF DESCRIPTION OF THE DRAWINGS

FIG. 1 illustrates a schematic diagram of the process for isolating HB α-2,6-SA-IVIG from human blood-derived mixed IVIG, and presents the results of immunoblotting analysis following the isolation. The loading amount was 1 µg. 1st Biotin-SNA was applied at 0.2 µg/ml for 30 minutes at room temperature, and 2nd SA-HRP was applied at 0.1 µg/ml for 30 minutes at room temperature. Upper bands corresponding to both light and heavy chains were observed in the elution and after-elution samples.
FIG. 2 demonstrates the high expression of DC-SIGN (CD209) mRNA in rabbit conjunctiva.
FIG. 3 illustrates the process of establishing a rabbit experimental model of dry eye syndrome.
FIG. 4 shows the increase in the anti-inflammatory cytokine IL-10 and the decrease in MMP9 and TGF-β on day 20 following administration of HB α-2,6-SA-IVIG in the rabbit dry eye syndrome model.
FIG. 5 depicts the method of evaluating eye damage using the non-invasive tear break-up time (NIBUT) test.
FIG. 6 illustrates the method of assessing ocular surface damage using vital staining techniques.
FIG. 7 shows that DC-SIGN (CD209) mRNA is highly expressed in the canine conjunctiva.
FIG. 8a shows the therapeutic effects of co-administration of HB α-2,6-SA-IVIG in canine clinical patients on corneal epithelial defects, keratitis, and keratoconjunctivitis sicca.
FIG. 8b shows the therapeutic effects of co-administration of HB α-2,6-SA-IVIG on blepharitis and keratoconjunctivitis sicca in canine clinical patients.
FIG. 8c shows the transient therapeutic effect of co-administration of HB α-2,6-SA-IVIG on chronic keratitis caused by posterior eye rupture in canine clinical patients.
FIG. 8d illustrates the therapeutic effect of co-administration of HB α-2,6-SA-IVIG on keratitis caused by corneal perforation induced by an ocular prosthesis in canine clinical patients.
FIG. 8e shows the therapeutic effects of co-administration of HB α-2,6-SA-IVIG on bilateral qualitative dry eye and chicken allergic keratoconjunctivitis in canine clinical patients.
FIG. 8f demonstrates the therapeutic effects of co-administration of HB α-2,6-SA-IVIG on epiphora, blepharitis, qualitative dry eye, and allergic conjunctivitis in canine clinical patients.
FIG. 8g illustrates the therapeutic effects of co-administration of HB α-2,6-SA-IVIG on keratitis, superficial corneal ulcer, and conjunctivitis in canine clinical patients.
FIG. 8h shows the therapeutic effect of co-administration of HB α-2,6-SA-IVIG on chronic corneal ulcer in canine clinical patients.

### BEST MODE FOR CARRYING OUT THE INVENTION

Unless otherwise defined, all technical and scientific terms used herein have the same meaning as commonly understood by one of ordinary skill in the art to which this invention belongs. The nomenclature used herein is generally well known and commonly employed in the relevant technical field.

In the present invention, it was confirmed that administration of α-2,6-sialylated immunoglobulin to rabbit models of dry eye syndrome and clinical canine subjects with inflammatory eye diseases effectively treated both dry eye syndrome and inflammatory eye conditions.

Specifically, α-2,6-sialylated immunoglobulin, when compared to administration of IVIG, significantly increased the expression level of the anti-inflammatory cytokine IL-10 in the eye, while not inducing fibrotic side effects in the corneal stroma. Thus, it demonstrated a remarkably advantageous effect over IVIG in the treatment of eye diseases. Notably, it was confirmed that α-2,6-sialylated immunoglobulin exerted a significant therapeutic effect even on refractory or severe inflammatory eye diseases that had not been responsive to conventional treatment methods.

Accordingly, in one aspect, the present invention relates to a pharmaceutical composition for preventing or treating dry eye syndrome and/or inflammatory eye diseases, comprising sialylated immunoglobulin.

In the present invention, the immunoglobulin may be derived from human blood and may be isolated from intravenous immunoglobulin (IVIG) or from lyophilized human immunoglobulin obtained through isolation and purification (e.g., https://www.pel-freez.com/human-igg-affinity-purified-lyophilized-34031), but it is not limited thereto.

As used herein, "intravenous immunoglobulin (IVIG)" refers to an injectable formulation of plasma-derived immunoglobulin containing antibodies against a wide range of pathogens, formulated to be suitable for systemic administration via intravenous injection.

Conventionally, immunoglobulins for therapeutic use were prepared for subcutaneous or intramuscular injection through methods such as cold ethanol fractionation. However, these routes of administration had several limitations, including restricted dosing capacity, low content of immunoglobulin G (IgG)-the key isotype responsible for antibody function-and degradation of immunoglobulin by proteases present at the injection site. To overcome these disadvantages of intramuscular administration, IVIG formulations suitable for intravenous injection have been developed by removing aggregates formed during manufacturing and storage, and by eliminating impurities such as blood coagulation factors (e.g., fibrinogen, albumin, PKA, transferrin) or other immunoglobulin isotypes (e.g., IgA and IgE), thereby improving the purity and stability of the product. Accordingly, the intravenous immunoglobulin of present invention encompasses not only IVIG products currently available on the market, but also newly developed or future formulations of IVIG without limitation.

Commercially available IVIG products include, but are not limited to, Carimune(Registered trademark), Flebogamma(Registered trademark), Gammagard(Registered trademark), Gammaked(Trademark name), Gammaplex, Gamunex(Registered trademark)-C, Octagam(Registered trademark), and Privigen(Registered trademark).

In the present invention, the sialylated immunoglobulin may include α-2,6-sialylated immunoglobulin. The α-2,6-sialylated immunoglobulin may be referred to by the abbreviation "HB α-2,6-SA-IVIG," which denotes an α-2,6-sialylated immunoglobulin isolated from IVIG (e.g., lyophilized IVIG or commercially available human IgG). However, in another embodiment, the α-2,6-sialylated immunoglobulin of the present invention may also be directly or indirectly isolated from human sources via alternative methods.

Furthermore, for the purpose of achieving the objects of the present invention, the α-2,6-sialylated immunoglobulin may be administered through routes other than intravenous injection.

In the present invention, the inflammatory eye disease may be an inflammatory eye disease of the eye, oscular surface, posterior segment of the eye or periocular region, but not limited thereto.

Specifically, the periocular region may include episclera, conjunctiva, eyelids, lacrimal glands, periocular glands, optic nerve, and the like, but not limited thereto.

In the present invention, the inflammatory eye disease may include at least one selected from the group consisting of hordeolum (stye), blepharitis, lid wiper epitheliopathy, chalazion, eyelid infection, conjunctivitis, anterior uveitis, conjunctival ulcer, iridocyclitis, scleritis, episcleritis, optic neuritis, retinal vasculitis, chronic vasculitis, posterior uveitis, pan uveitis, meibomian gland dysfunction (MGD), and dacryoadenitis, but not limited thereto.

In the present invention, the inflammatory eye disease may be induced by allergic responses, by conventional treatments such as steroid injections, or by surgical procedures or physical stimuli including ocular implants.

The α-2,6-sialylated immunoglobulin, or a pharmaceutical composition comprising the same, may, in one embodiment, be administered as a monotherapy for treating dry eye syndrome or inflammatory eye diseases. In another embodiment, it may be co-administered with other therapeutic agents for treating dry eye syndrome or inflammatory eye diseases.

The other therapeutic agents for treating dry eye syndrome and/or inflammatory eye diseases may include, but are not limited to, at least one selected from the group consisting of an anti-inflammatory agent, an immunosuppressant, an anti-glaucoma agent, artificial tear, tear secretion stimulant, and an antibiotics.

In the present invention, the other therapeutic agents for treating dry eye syndrome and/or inflammatory eye diseases may include, but are not limited thereto, Optimmune^{®} (0.2% cyclosporine), an-Hypro (1.2% hyaluronate eye gel), Maxitrol^{®} (dexamethasone/neomycin sulfate/polymyxin b sulfate), Fumelon^{®} (fluorometholone), Tarivid^{®} (ofloxacine), Hamerone-P (0.3% sodium hyaluronate), Levofloxacin, Diquas, Fumelon, Chloramphenicol, and hyaluronic acid (0.3%), among others. In such cases, the dosage of the therapeutic agents may follow the manufacturer's recommended dose or may be appropriately adjusted by a clinician (physician or veterinarian) based on the condition of the subject to be treated.

**In** another embodiment of the present invention, the α-2,6-sialylated immunoglobulin or a pharmaceutical composition comprising the same may be administered in combination with a surgical procedure or surgical operation.

**In** the present invention, the pharmaceutical composition may be in the form of an ophthalmic solution, although it is not limited thereto.

**In** the present invention, the α-2,6-sialylated immunoglobulin, although not limited thereto, may be characterized in that, as compared to conventional intravenous immunoglobulin (IVIG), it (i) enhances the expression of IL-10, an anti-inflammatory cytokine in ocular cells, by 50% or more and/or (ii) does not induce fibrotic side effects in the corneal stroma. Due to these characteristics, the α-2,6-sialylated immunoglobulin may exhibit superior therapeutic effects in the treatment of dry eye syndrome and/or inflammatory eye diseases compared to conventional IVIG.

The pharmaceutical composition of the present invention may further comprise various excipients including pharmaceutically acceptable diluents or carriers. In some embodiments, the pharmaceutical composition of the present invention may be provided in a method for administration to a subject as needed. In certain embodiments, the pharmaceutical composition of the present invention may be administered to humans.

The pharmaceutical composition of the present invention may be administered to a patient either alone or in combination with other agents. The term "co-administration" refers to simultaneous or sequential administration individually or in combination with one or more other drugs. Therefore, the pharmaceutical preparation of the present invention may also be formulated as a single formulation together with one or more other drugs.

The pharmaceutical composition of the present invention may be formulated using conventional methods in the form of oral dosage forms such as powders, granules, tablets, capsules, suspensions, emulsions, syrups, or aerosols; topical preparations; suppositories; or sterile injectable solutions.

For example, the pharmaceutical composition of the present invention may be formulated as an injection, ointment, gel, lotion, capsule, tablet, solution, suspension, spray, inhalant, eye drop, patch, or adhesive preparation.

The pharmaceutical composition of the present invention may be administered orally or parenterally(such as intravenous, subcutaneous, intramuscular, intraperitoneal, intranasal, vaginal, intrathecal, intra-articular, or topical administration) depending on the intended method.

Solid formulations for oral administration may include tablets, pills, powders, granules, and capsules. These solid formulations may be prepared by mixing with at least one excipient such as starch, calcium carbonate, sucrose, lactose, or gelatin. In addition to simple excipients, lubricants such as magnesium stearate and talc may also be used.

Liquid formulations for oral administration may include suspensions, internal solutions, emulsions, and syrups, and may further comprise various excipients such as wetting agents, sweeteners, flavoring agents, and preservatives, in addition to simple diluents such as water or liquid paraffin.

Formulations for parenteral administration may include sterile aqueous solutions, non-aqueous solvents, suspensions, emulsions, lyophilized formulations, and suppositories. Suitable non-aqueous solvents and suspensions include injectable esters such as propylene glycol, polyethylene glycol, vegetable oils such as olive oil, and ethyl oleate.

The pharmaceutical composition may be a sterile injectable preparation in the form of a sterile aqueous or oily suspension. Such a suspension may be formulated using suitable dispersing or wetting agents (e.g., Tween 80) and suspending agents according to techniques well known in the art. The sterile injectable preparation may also be a sterile injectable solution or suspension in a nontoxic parenterally acceptable diluent or solvent (e.g., a solution in 1,3-butanediol). Suitable vehicles and solvents include glycine, proline, sorbitol, maltose, sucrose, mannitol, water, Ringer's solution, and isotonic sodium chloride solution. Sterile non-volatile oils may also be commonly used as solvents or suspension media. For this purpose, any non-irritating non-volatile oil, including synthetic mono- or diglycerides, may be used. Natural oils (e.g., olive oil or castor oil), particularly their polyoxyethylated derivatives, as well as fatty acids such as oleic acid and glyceride derivatives thereof, may be useful for injectable formulations.

The pharmaceutical composition of the present invention may also be administered in the form of a suppository for rectal administration. Such compositions may be prepared by mixing the compound of the present invention with a suitable non-irritating excipient that is solid at room temperature but liquefies at rectal temperature. Examples of such excipients include, but are not limited to, cocoa butter, beeswax, and polyethylene glycol.

Parenteral administration of the pharmaceutical composition of the present invention is particularly useful when the intended treatment involves a site or organ that is accessible by local application. When applied topically to the skin, the pharmaceutical composition should be formulated as a suitable ointment containing the active ingredient suspended or dissolved in a carrier. Suitable carriers for topical application of the compound of the present invention include, but are not limited to, mineral oil, liquid paraffin, white petrolatum, propylene glycol, polyoxyethylene, polyoxypropylene compounds, emulsifying wax, and water. In another embodiment, the pharmaceutical composition of the present invention may be formulated as a suitable lotion or cream containing the active compound suspended or dissolved in a carrier. Suitable carriers include, but are not limited to, mineral oil, sorbitan monostearate, polysorbate 60, cetyl ester wax, cetearyl alcohol, 2-octyldodecanol, benzyl alcohol, and water. The pharmaceutical composition of the present invention may also be administered rectally via suppositories or as an appropriate enema formulation for local delivery to the lower gastrointestinal tract. Topical transdermal patches are also included within the scope of the present invention.

The pharmaceutical composition of the present invention may be administered via nasal aerosol or inhalation. Such compositions may be prepared as solutions in saline using techniques well known in the art, and may comprise benzyl alcohol or other suitable preservatives, absorption enhancers to increase bioavailability, fluorocarbons, and/or other solubilizing or dispersing agents known in the field.

The relative amounts of active ingredients, pharmaceutically acceptable excipients, and/or any additional ingredients in the pharmaceutical composition of the present invention will vary depending on the identity, size, and/or condition of the subject being treated, as well as the route of administration. For example, the content of the active ingredient in the pharmaceutical composition of the present invention is not particularly limited, but may be included in an amount of 0.0001 to 100% by weight, preferably 0.001 to 50% by weight, and more preferably 0.01 to 10% by weight, based on the total weight of the final composition.

In the present invention, the pharmaceutically acceptable excipient may include any solvent, dispersion medium, diluent, or other liquid vehicle, dispersion or suspension aid, surfactant, isotonic agent, thickener or emulsifier, preservative, solid binder, lubricant, or the like, suitable for the intended dosage form. The literature Remington [The Science and Practice of Pharmacy, 21st Edition, A. R. Gennaro (Lippincott, Williams & Wilkins, Baltimore, MD, 2006)] describes various excipients and known techniques used for preparing pharmaceutical compositions.
Unless otherwise incompatible due to causing undesirable biological effects or harmful interactions with other components of the composition, any conventional carrier medium is considered within the scope of the present invention. The pharmaceutically acceptable excipient is preferably at least 95%, 96%, 97%, 98%, 99%, or 100% pure.

The excipients are approved for human and veterinary use. In some embodiments, the excipients are approved by the U.S. Food and Drug Administration (FDA). In certain embodiments, the excipients are of pharmaceutical grade. In some embodiments, the excipients meet the standards of the United States Pharmacopeia (USP), European Pharmacopeia (EP), British Pharmacopeia (BP), and/or International Pharmacopeia.

The term "prevention" as used in the present invention includes preventing or delaying the onset of clinical or subclinical symptoms of a condition, disorder, or disease in a subject, particularly a mammal, who may be susceptible to such condition, disorder, or disease but has not yet experienced or exhibited its clinical or subclinical symptoms.

The term "treatment" as used in the present invention includes:
(1) suppressing a condition, disorder, or disease (e.g., inhibiting the onset of the disease, or in the case of maintenance therapy, suppressing, reducing, or delaying the recurrence of at least one clinical or subclinical symptom); and/or
(2) alleviating a condition, disorder, or disease (i.e., causing regression of the condition, disorder, or disease, or at least one clinical or subclinical symptom thereof).

The benefit to the patient receiving treatment may be statistically significant, or at the very least, perceptible by the patient or physician. However, those skilled in the art will understand that administration of a drug to treat a disease does not always result in effective treatment.

The pharmaceutical compositions provided in the present invention are, in principle, intended for administration to humans; however, one skilled in the art will understand that such compositions are generally suitable for administration to all types of animals. That is, the pharmaceutical compositions of the present invention may also be administered to animals in need of veterinary treatment, such as companion animals (e.g., dogs, cats), livestock (e.g., cattle, sheep, pigs, horses), and laboratory animals (e.g., rats, mice, guinea pigs), among other mammals. Variants of the pharmaceutical compositions for use in different animal species are well understood, and a skilled veterinary pharmacologist can readily design and/or carry out such variants through routine experimentation if necessary.

The pharmaceutical compositions described herein may be prepared by any method known in the field of pharmacology or as described in subsequent sections. In general, such preparation methods include the step of associating the active ingredient with one or more excipients and/or auxiliary substances, followed by a step of formulating and/or packaging the resulting product into a desired single or multi-dose unit, if necessary or desired.

The pharmaceutical composition of the present invention may be prepared, packaged, and/or sold whether packaged or unpackaged in the form of a single unit dose and/or multiple unit doses. As used herein, the term "unit dose" refers to a discrete amount of the pharmaceutical composition comprising a predetermined quantity of the active ingredient. The amount of active ingredient is generally equal to the dosage to be administered to the subject or a convenient fraction thereof, such as one-half or one-third of the total dosage.

The term "administration" in the present invention refers to introducing a given substance to a patient by any suitable means. The route of administration of the pharmaceutical composition is not limited so long as the drug can reach the target tissue. The route of administration may include, but is not limited to, intraperitoneal, intravenous, intramuscular, subcutaneous, intradermal, oral, topical, intranasal, intrapulmonary, rectal, vaginal, intrathecal, or intra-articular administration.

The pharmaceutical composition of the present invention may be administered in a pharmaceutically effective amount.

The term "pharmaceutically effective amount," as used herein, may refer to a "therapeutically effective amount," which is an amount of a compound or composition (e.g., a compound or composition of the present invention) sufficient to achieve a beneficial or desired result. A pharmaceutically effective amount may be administered in one or more doses, applications, or administrations, and is not intended to be limited to a particular formulation or route of administration.

In the present invention, the daily dosage of HB α-2,6-SA-IVIG may be approximately 0.01 µg to 100 µg, preferably about 0.1 µg to 20 µg.

The HB α-2,6-SA-IVIG according to the present invention exhibited a significant therapeutic effect on canine eye diseases when administered via ocular instillation at a dose of 5 µg/100 µL per day (2.5 µg/50 µL per administration, twice daily; formulated in PBS). However, since α-2,6-SA-IVIG is derived from human immunoglobulin, it may have a higher binding affinity for human DC-SIGN, and when administered to humans, it shows significantly improved signaling and IL-10 immunosuppressive cytokine production (Clin Immunol. 2023 Jan; 246:109215. doi: 10.1016/j.clim.2022.109215).

Therefore, it is expected that for human eye diseases, HB α-2,6-SA-IVIG may exhibit therapeutic effects for dry eye syndrome and inflammatory eye diseases at even lower daily doses, for example, approximately 0.1 to 3 µg, preferably 0.1 to 1 µg.

However, the effective dosage level may vary depending on the severity of the disease, the activity of the drug, the age, weight, health, sex, and drug sensitivity of the patient, the timing, route of administration, excretion rate, duration of treatment, and other concomitant drugs or formulations used in combination with the composition of the present invention, as well as other factors well known in the medical field.

The pharmaceutical composition of the present invention may be administered as a monotherapy or in combination with other therapeutic agents, and may be administered sequentially or simultaneously with conventional therapies. It may be administered as a single dose or in multiple doses. Considering all of the above factors, it is important to administer the minimum amount necessary to achieve maximum effect without adverse effects.

The dosage of the pharmaceutical composition of the present invention may be determined by a person skilled in the art based on the intended use, the severity of the disease, the age, weight, sex, and medical history of the patient, or the nature of the active substance being used.

From another perspective, the present invention relates to an ophthalmic composition comprising α-2,6-sialylated immunoglobulin.

In the present invention, the ophthalmic composition may be in the form of a solution (e.g., aqueous liquid formulation) or ointment, but is not limited thereto.

The ophthalmic composition may be formulated as a solution, ointment, or gel.

In one embodiment, the ophthalmic composition may be an artificial tear composition.

From another perspective, the present invention relates to a method for preventing or treating dry eye syndrome or inflammatory eye disease, comprising administering α-2,6-sialylated immunoglobulin or the pharmaceutical composition comprising the same to a subject in need thereof.

In the present invention, the subject may be a human or a non-human animal.

In the present invention, the α-2,6-sialylated immunoglobulin or the pharmaceutical composition may be administered simultaneously, separately, or sequentially with other therapeutic agent for treating dry eye syndrome or inflammatory eye disease.

From another perspective, the present invention relates to the use of α-2,6-sialylated immunoglobulin or the pharmaceutical composition for preventing or treating dry eye syndrome or inflammatory eye diseases.

From yet another perspective, the present invention relates to the use of α-2,6-sialylated immunoglobulin or the pharmaceutical composition in the preparation of a medicament for preventing or treating dry eye syndrome or inflammatory eye diseases.

For the above therapeutic methods and uses, unless otherwise contradictory, the description provided for the pharmaceutical composition shall be equally applicable.

As used herein, the terms "individual", "patient", and "subject" are used interchangeably and include any animal, such as mammals including, for example, mice, rats, other rodents, rabbits, dogs, cats, pigs, cattle, sheep, horses, or primates including humans.

As used in this specification, the term "about" or "approximately" refers to being within an acceptable deviation range from the specified value as determined by one skilled in the art, depending in part on how the value is measured or determined, i.e., based on the limitations of the measurement system. For example, "about" may mean within one or more standard deviations of a given value as practiced in the art. Alternatively, "about" may mean within 20%, 10%, 5%, or 1% of a given value or range. In some cases, particularly in the context of biological systems or processes, the term may mean within a magnitude of 2-fold or 5-fold of the specified value. Unless otherwise stated, when a specific value is recited herein or in the claims, the term "about" is intended to encompass variations within the acceptable deviation range of that value.

### Example

The following examples are provided to further illustrate the present invention in detail. These examples are intended solely for illustrative purposes and are not to be construed as limiting the scope of the invention in any way, as it will be apparent to those skilled in the art.

### Example 1. Isolation of HB α-2,6-SA-IVIG from IVIG

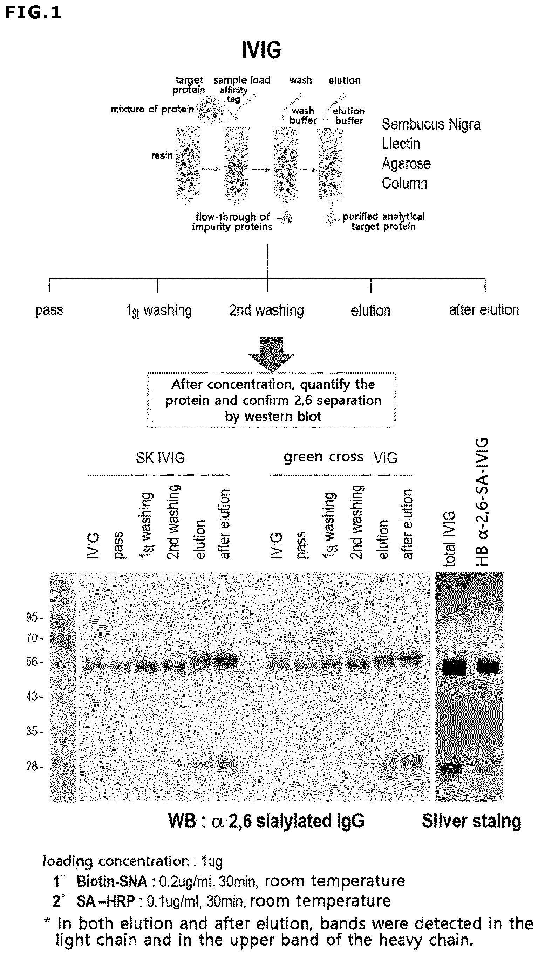

The commercially available IVIG preparations used in clinical practice are mixtures containing various forms of immunoglobulins (e.g., IgG1, IgG2, IgG3) with more than 300 different types of glycosylation patterns. The present inventors established a method for isolating only HB α-2,6-SA-IVIG from such mixed-form IVIG (FIG. 1).

The sources of materials used in the experiment were as follows: SNA lectin (Vector, Cat.#: AL-1303), Econo-Column (Bio-Rad, Cat.#: 7371007). The wash buffer consisted of 1X TBS and 0.1 mM CaCl₂ and was stored at 4°C after filtration. Elution buffer ① (0.5 M lactose dissolved in TBS) and elution buffer ② (0.5 M lactose dissolved in acetic acid) were prepared by dissolving lactose at 56°C and then cooling at -20°C or -80°C. The agarose-bound SNA lectin was washed with TBS prior to use, and 2 mL of the washed agarose-bound SNA lectin was added to the column. IVIG was diluted to 20 mg/2 mL using TBS and 0.1 mM CaCl₂. The diluted IVIG (20 mg/2 mL) was then applied to the agarose-bound SNA lectin, ensuring that the beads to float in suspension. The mixture was incubated at room temperature for 10 minutes, after which the flow-through (2 ml, 2,6-SA(-)-IVIG) was collected. The collected material was washed with 4 mL of TBS and 0.1 mM CaCl2 by gently adding the buffer to avoid bead suspension, and the flow-through was collected. Then, another 4 mL of TBS and 0.1 mM CaCl₂ was gently added in the same manner to avoid bead suspension, and the resulting flow-through was collected, and a second wash was performed. Thereafter, 2 mL of elution buffer ① (0.5 M lactose in TBS) was added, followed by incubation at room temperature for 10 minutes. The suspension was added to the column, ensuring that the beads to float in suspension, and the eluate was collected. Subsequently, 2 mL of elution buffer ② (0.5 M lactose in 0.2 M acetic acid) was added, incubated at room temperature for 10 minutes, and the eluate was collected in the same manner. Finally, the column was washed several times with 4 mL of TBS and 0.1 mM CaCl₂.

The fraction was dialyzed against PBS and concentrated using a centrifugal filtration device (10 kDa, Amicon, Millipore). PBS, in an amount three times the volume of the fraction, was added, and the mixture was concentrated until the volume reached approximately 1 mL (2000 rcf, 4°C). Thereafter, 10 mL of PBS was added and the sample was further concentrated until the final volume reached 0.5 mL (2000 rcf, 4°C).

From the mixed-form IVIG derived from human blood purchased from SK and Green Cross, HB α-2,6-SA-IVIG was isolated through the procedure described above. At each step of the isolation, the sialylation of HB α-2,6-SA-IVIG was confirmed by Western blot using biotinylated polymeric Sambucus Nigra Lectin (SNA) (Vector, USA). As a result, it was confirmed that sialylated HB α-2,6-SA-IVIG was collected only in the elution and after-elution fractions from the IVIG raw materials of both companies (bands of both the heavy chain and light chain of HB α-2,6-SA-IVIG were observed to be larger in size than those of IVIG).

### Example 2. Confirmation of Therapeutic Effect in a Rabbit Model of Dry Eye Syndrome

### 2-1. Confirmation of DC-SIGN Expression in Rabbit Conjunctiva

Conjunctival tissue, as well as liver, spleen, and lymph nodes, were collected from rabbits (Koatech, Korea). Total RNA was extracted using RNAiso Plus (Takara) in accordance with the manufacturer's protocol. The isolated RNA was quantified and subsequently used for cDNA synthesis using the High-Capacity cDNA Reverse Transcription Kit (appliedbiosystems). For PCR, 1 µL of the synthesized cDNA was mixed with 0.2 mM of each gene-specific primer, and amplification was carried out using Platinum TM II Taq Hot-Start DNA Polymerase. PCR conditions were as follows: 94°C for 15 seconds, 60°C for 15 seconds, and 68°C for 15 seconds, repeated for 35 cycles. The resulting PCR products were electrophoresed on a 2% agarose gel and stained with ethidium bromide to confirm the expression levels of each gene.

Rabbit DC-SIGN amplification primers:
   F: 5'-TGTGCTTCACGCTGTTCACT-3'
   R: 5'-CTCCTCAGCACTTTGGACGA-3'
Rabbit GAPDH amplification primers:
   F: 5'-TGACGACATCAAGAAGGTGGTG-3'
   R: 5'-GAAGGTGGAGGAGTGGGTGGC-3'

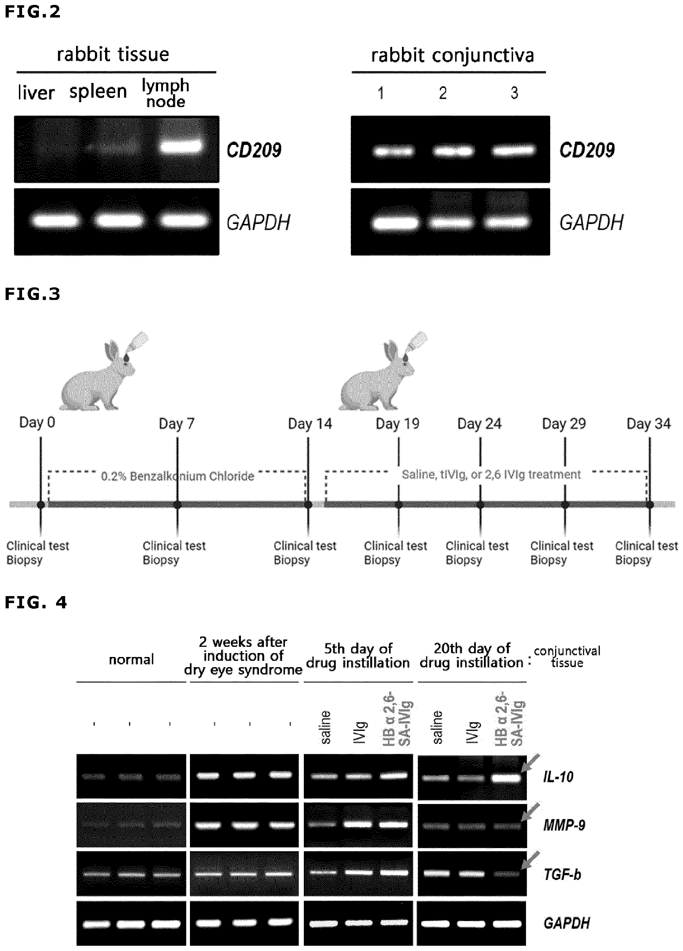

As a result, it was confirmed that DC-SIGN is expressed not only in the spleen and lymph nodes but also in the conjunctival tissue of rabbits (FIG. 2).

### 2-2. Establishment of Dry Eye Syndrome Rabbit Model and Drug Administration

A rabbit model of dry eye syndrome was established by topical administration of 0.2% benzalkonium chloride to the eyes of rabbits (FIG. 3).

Subsequently, the rabbits were randomly divided into three groups of five animals each: control group (0.9% normal saline), test group 1 (0.4% IVIG, 4 mg/mL in 0.9% normal saline), and test group 2 (0.04% HB α-2,6-SA-IVIG, 4 mg/mL in 0.9% normal saline). Each eye drop formulation was administered topically twice daily for 20 consecutive days. In all groups, antibiotic eye drops were also administered. The volume of each dose was 100 µL (IVIG: 400 µg, α-2,6-SA-IVIG: 40 µg). The total dosage administered was 16 mg of IVIG (0.8 mg/200 µL per day × 20 days) and 1.6 mg of HB α-2,6-SA-IVIG (0.08 mg/200 µL per day × 20 days).

The corneas of the rabbits were collected on days 0, 7, 14, 19, 24, 29, and 34 (one sample area per collection: 0.15 mm²).

### 2-3. Analysis of Anti-inflammatory Cytokine Induction by α-2,6-SA-IVIG

To evaluate the anti-inflammatory effect of HB α-2,6-SA-IVIG, HB α-2,6-SA-IVIG was administered to the dry eye syndrome rabbit model, and changes in the expression levels of anti-inflammatory cytokines IL-10, MMP-9, and TGF-β were analyzed.

Total RNA was extracted from the corneal tissue collected in Example 2-2 using RNAiso Plus (Takara), following the manufacturer's instructions. The isolated RNA was quantified and used to synthesize cDNA with the High-Capacity cDNA Reverse Transcription Kit (appliedbiosystems). For PCR, 1 µL of synthesized cDNA was mixed with 0.2 mM of each gene-specific primer, and amplification was performed using Platinum TM II Taq Hot-Start DNA Polymerase. The PCR conditions were as follows: 94°C for 15 seconds, 60°C for 15 seconds, and 68°C for 15 seconds, repeated for 35 cycles. The resulting PCR products were electrophoresed on a 2% agarose gel, stained with ethidium bromide, and the expression levels of each gene were evaluated.

Rabbit IL-10 amplification primers
   F: 5'-GAGAACCACAGTCCAGCCAT-3'
   R: 5'-CATGGCTTTGTAGACGCCTT-3'
Rabbit MMP-9 amplification primers
   F: 5'-GAGTACCTGTTCCGCTATG-3'
   R: 5'-TGCCACTTGAGGTCACCCTCGAA-3'
Rabbit TGF-β amplification primers
   F: 5'-TGGCTGAACAACACATAGAACTG-3'
   R: 5'-ACGCAGGCAGCAATTATCCT-3'

As a result, administration of IVIG in the rabbit model of dry eye syndrome did not lead to an increase in IL-10 compared to the control group. In contrast, administration of HB α-2,6-SA-IVIG resulted in an increase in IL-10 by day 5 of eye drop treatment, even at one-tenth the dose of IVIG. By day 20, IL-10 expression was significantly elevated, indicating that HB α-2,6-SA-IVIG exerts a potent anti-inflammatory effect.

Meanwhile, MMP-9 present in tears promotes desquamation of corneal and conjunctival epithelial cells and induces epithelial erosion of the cornea and conjunctiva. Thus, an initial increase in MMP-9 is beneficial for the removal of damaged epithelial cells caused by dry eye syndrome, but if the erosion becomes chronic, it can lead to keratoconjunctivitis; therefore, MMP-9 levels should decrease during the latter phase of treatment. In the dry eye syndrome rabbit model, both IVIG and HB α-2,6-SA-IVIG treatment resulted in increased MMP-9 expression by day 5. However, by day 20, MMP-9 levels decreased to near-normal levels, confirming that both agents are suitable for the treatment of dry eye syndrome.

TGF-β is a molecule secreted into tears in response to damage to the corneal stroma. While it plays a role in maintaining corneal integrity and promoting wound healing, it also induces stromal fibrosis, leading to scar formation in the cornea. In the dry eye syndrome rabbit model, IVIG treatment resulted in sustained elevation of TGF-β levels on both day 5 and day 20. In contrast, HB α-2,6-SA-IVIG treatment caused an increase in TGF-β expression only on day 5, followed by a sharp decrease by day 20. These findings suggest that HB α-2,6-SA-IVIG is particularly suitable as a therapeutic agent for the treatment of dry eye syndrome (FIG. 4).

### 2-4. Confirmation of Therapeutic Effect of HB α-2,6-SA-IVIG on Dry Eye Syndrome Using NIBUT Method

As in Example 2-2, eye drops were administered in a rabbit model of dry eye syndrome, and on days 5, 10, 15, and 20 of treatment, the therapeutic effect was assessed using the NIBUT (Non-Invasive Break-Up Time) mode of the OSA-VET system (SBM Sistemi). After a single blink, the rabbit's eye was held open to prevent blinking.

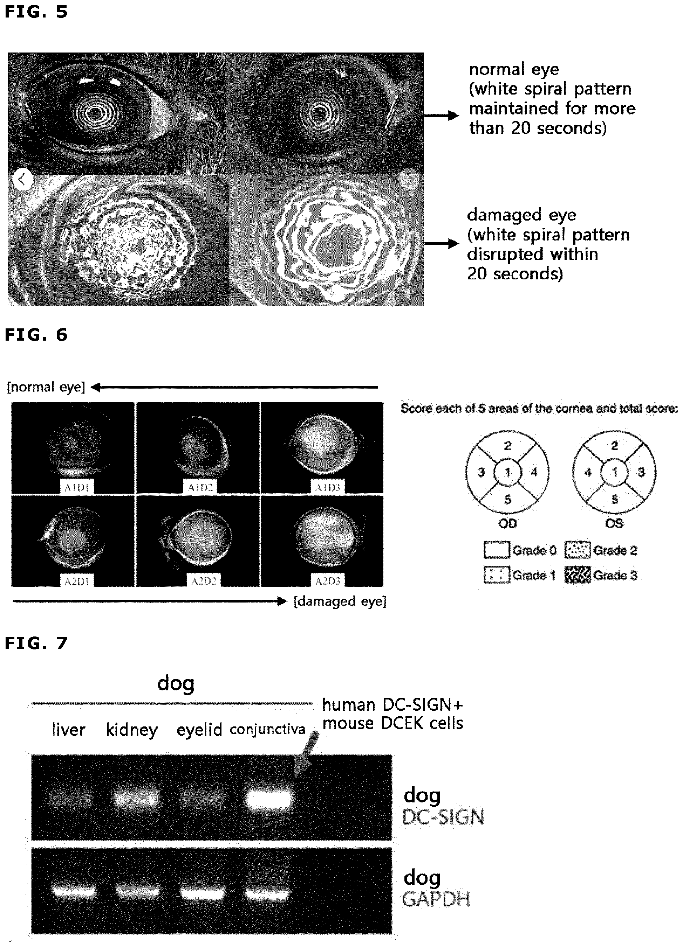

NIBUT (Non-Invasive Break-Up Time) projects concentric rings of light onto the eye surface and measures and analyzes the position and timing at which these rings become irregular. A stable white spiral ring maintained for more than 20 seconds was classified as normal, whereas distortion of the white spiral pattern within 20 seconds was diagnosed as damaged (FIG. 5).

**[Table 1]**

| Confirmation of Eye Recovery Result by NIBUT Method | | | |
|---|---|---|---|
| Ocular instillation of the therapeutic drug | 0.9% normal saline | IVIG 0.4% | α-2,6-SA-IVIG |
| Day 5 | 4 eyes | 5 eyes | 4 eyes |
| Day 10 | 4 eyes | 2 eyes | 6 eyes |
| Day 15 | 1 eye | 3 eyes | |
| Day 20 | - | | |
| Eyes that did not recover by day 20 | 1 eye | | |

As a result of the experiment, in the dry eye syndrome rabbit model, administration of IVIG led to full recovery of all 10 eyes within 15 days. In contrast, administration of HB α-2,6-SA-IVIG at one-tenth the dose of IVIG resulted in full recovery of all 10 eyes within just 10 days (Table 1).

### 2-5. Confirmation of the Therapeutic Effect of HB α-2,6-SA-IVIG on Dry Eye Syndrome Using the Vital Staining Method

The vital staining method is a technique for evaluating corneal epithelial damage using fluorescein dye. In the dry eye syndrome rabbit model, as described in Example 2-2, vital staining was performed on days 5, 10, 15, and 20 following drug instillation.

A single drop of normal saline was applied to a fluorescein strip, and the strip was gently contacted with the cornea of the rabbit model to allow staining of the corneal surface with the dye. Afterward, the dye was carefully rinsed off with approximately 5 mL of normal saline, and the stained areas of the cornea were examined using a slit lamp microscope. In normal eyes, the fluorescein dye does not stain the corneal surface, whereas in damaged eyes, the extent of corneal staining by fluorescein dye increases (FIG. 6).

As a result of the experiment, in the dry eye syndrome rabbit model, all 10 eyes fully recovered within 15 days following IVIG administration. Similarly, administration of HB α-2,6-SA-IVIG at one-tenth the dose of IVIG also led to full recovery of all 10 eyes within 15 days (Table 2).

**[Table 2]**

| Confirmation of Eye Recovery(No Fluorescein Staining) Result by Vital Staining Method | | | |
|---|---|---|---|
| Ocular instillation of the therapeutic drug | 0.9% normal saline | IVIG 0.4% | α-2,6-SA-IVIG |
| Day 5 | 0 eye | 0 eye | 1 eye |
| Day 10 | 0 eye | 3 eyes | 1 eye |
| Day 15 | 5 eyes | 6 eyes | 8 eyes |
| Day 20 | 4 eyes | 1 eye | |
| Eyes that did not recover by day 20 | 1 eye | | |

### Example 3. Evaluation of the Therapeutic Efficacy in Canine Inflammatory Eye Diseases

### 3-1. Confirmation of DC-SIGN Expression in Canine Conjunctiva

Conjunctiva, liver, kidney, and eyelid tissues were collected from a canine (Jabio, Korea), and total RNA was extracted using RNAiso Plus (Takara) according to the manufacturer's instructions. The extracted RNA was quantified and used for cDNA synthesis using the High-Capacity cDNA Reverse Transcription Kit (appliedbiosystems). For PCR, 1 µL of synthesized cDNA was mixed with 0.2 mM of each gene-specific primer, and amplification was performed using Platinum TM II Taq Hot-Start DNA Polymerase. The PCR conditions were as follows: 94°C for 15 seconds, 60°C for 15 seconds, and 68°C for 15 seconds, repeated for 35 cycles. The resulting PCR products were electrophoresed on a 2% agarose gel and stained with ethidium bromide to evaluate the expression level of each gene.

Canine DC-SIGN amplification primers:
   F: 5'-CTCCCAGACCCAAAACACCT-3'
   R: 5'-CTGGCCTTGGGGAGCAAAAG-3'
Canine GAPDH amplification primers:
   F: 5'-GTCAAGGCTGAGAACGGGAA-3'
   R: 5'-CTCCGATGCCTGCTTCACTA-3'

As a result, DC-SIGN was found to be expressed in the liver, kidney, eyelid, and conjunctival tissues of the dog, with expression levels being particularly high in the conjunctival tissues (FIG. 7).

For reference, a PCR experiment was also conducted using the DCEK mouse cell line (Rockefeller University, USA, obtained under a material transfer agreement (MTA)), which highly expresses human DC-SIGN. It was confirmed that the canine DC-SIGN PCR primers did not recognize human DC-SIGN at all (see the final lane in FIG. 7), thereby additionally confirming that the canine DC-SIGN primers specifically recognize only canine DC-SIGN.

### 3-2. Confirmation of Therapeutic Effect in Clinical Subjects with Canine Inflammatory Eye Diseases

To verify the therapeutic effect of HB α-2,6-SA-IVIG, which has been proven effective and non-toxic in the treatment of dry eye syndrome in the rabbit model, dogs diagnosed with inflammatory eye diseases were recruited from a secondary veterinary ophthalmology hospital.

The enrolled canine subjects met the following criteria: (1) Cases referred to the secondary ophthalmology hospital due to lack of response to conventional treatments at local veterinary clinics, where further treatment attempts at the secondary hospital also failed, (2) Cases referred to the secondary ophthalmology from local clinics after failure of conventional therapies, in which the eye condition was too severe at the time of referral to allow further application of conventional treatments, (3) Cases directly referred to the secondary ophthalmology hospital due to severe eye disease, where conventional treatment had already been attempted but was ineffective. Detailed case descriptions are summarized in Table 3.

**[Table 3]**

| No | species | symptom | Prescription (Treatment at Local Animal Hospital) |
|---|---|---|---|
| 1 | Boston Terrier | - Bilateral corneal epithelial defects andkeratitis | - Optimmune^{®} (0.2% Cylcosporine) |
| | | - right eye with keratoconjunctivitis sicca | - an-Hypro(1.2% Hyaluronate eye gel) |
| 2 | Maltese | - Bilateral blepharitis, keratoconjunctivitis sicca | - Maxitrol^{®} (dexamethasone/neomyc in sulfate/polymyxin b sulfate) |
| | | | - Optimmune^{®} (0.2% Cylcosporine) |
| | | | - an-Hypro(1.2% Hyaluronate eye gel) |
| 3 | Pomera nian | - Posterior rupture of the right eye(Day 0) | - Maxitrol^{®} (dexamethasone/neomyc in sulfate/polymyxin b sulfate) |
| | | - enucleation followed by prosthetic eyeimplantation(Day 1) | |
| | | - chronic keratitis in the right eye(Day 60) | - Optimmune^{®} (0.2% Cylcosporine) |
| 4 | Poodle | - Malignant glaucoma in the right eye diagnosed, followed by enucleation and prosthetic eye implantation (Day 0) | - Prosthetic eye implantation |
| | | | - Optimmune^{®} (0.2% Cyclosporine) |
| | | - Corneal perforation and severe keratitis in the right eye (Day 86) | |
| 5 | Pomera nian | - Bilateral pigmentary keratitis and severe chronic keratoconjunctivitis sicca | - Maxitrol^{®} (dexamethasone/neomyc in sulfate/polymyxin b sulfate) |
| | | | - Optimmune^{®} (0.2% Cylcosporine) |
| 6 | Maltese | - Bilateral qualitative dry eye | - Fumelon^{®} (Fluorometholone) |
| | | | - an-Hypro (1.2% Hyaluronate eye gel) |
| 7 | French Bulldog | - Keratohelcosis | - Direct referral to the secondary veterinary hospital without prior treatment at a local clinic |
| 8 | Boston Terrier | - Recurrent chronic keratohelcosis | - Tarivid^{®} (Ofloxacine) |
| | | | - Hamerone-P (0.3% sodium hyaluronate) |
| | | | - an-Hypro (1.2% Hyaluronate eye gel) |

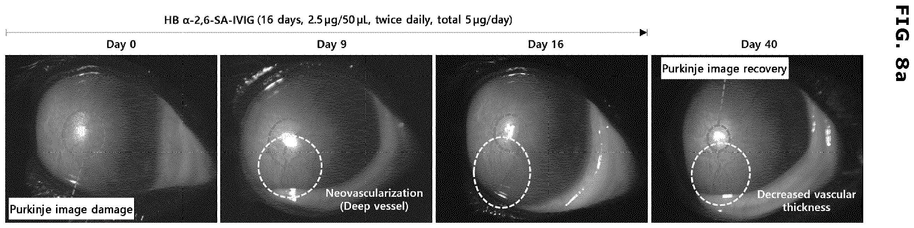

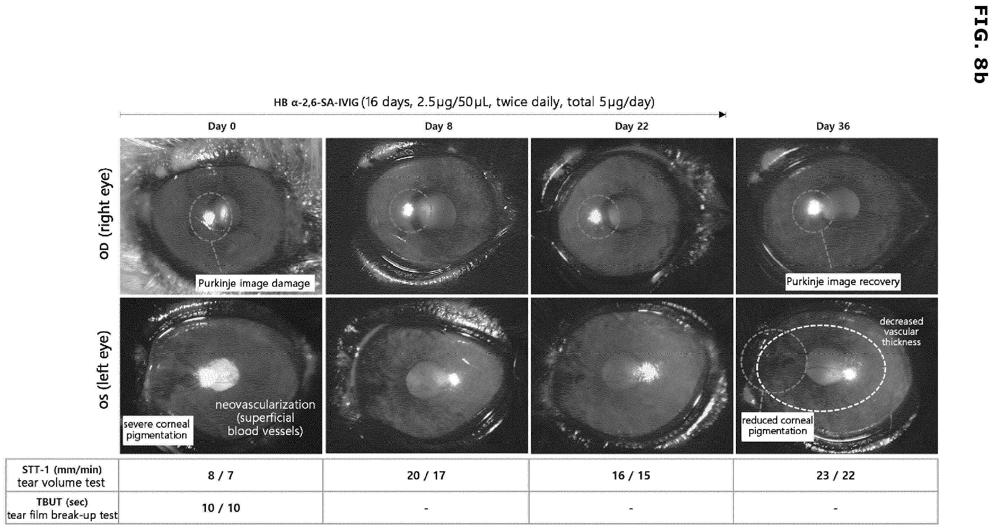

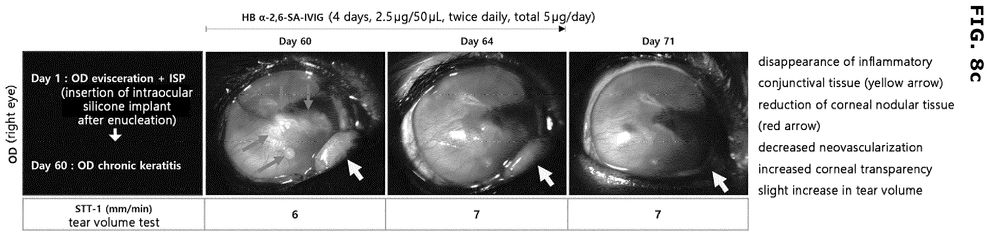

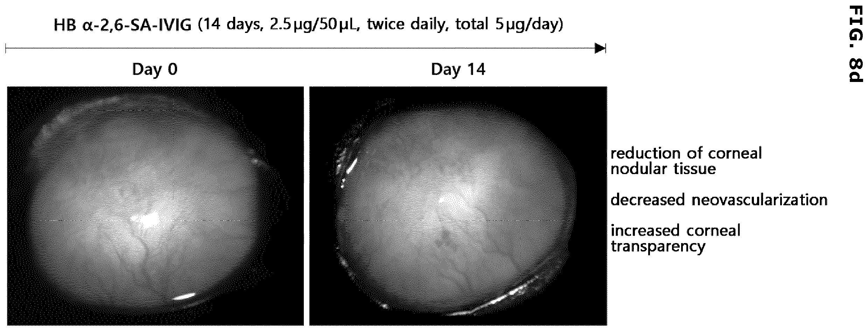

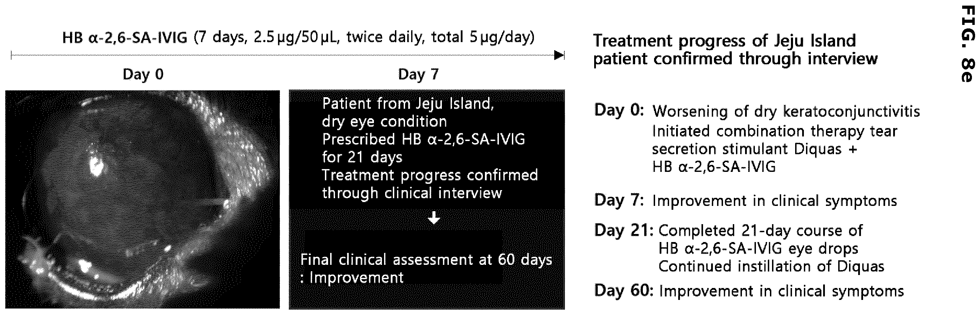

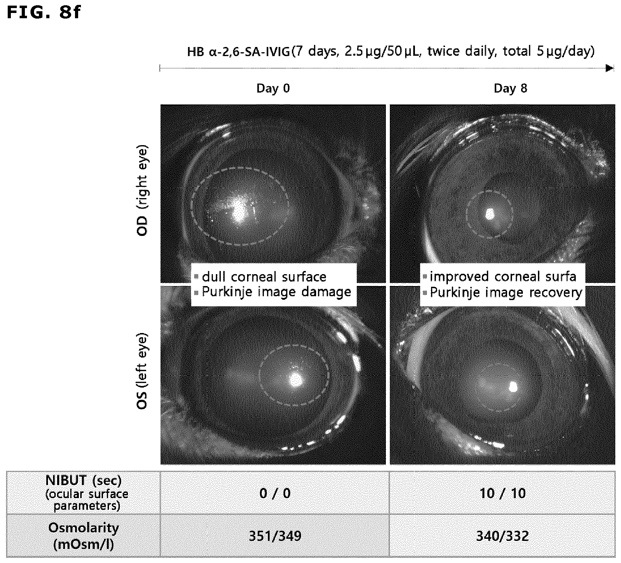

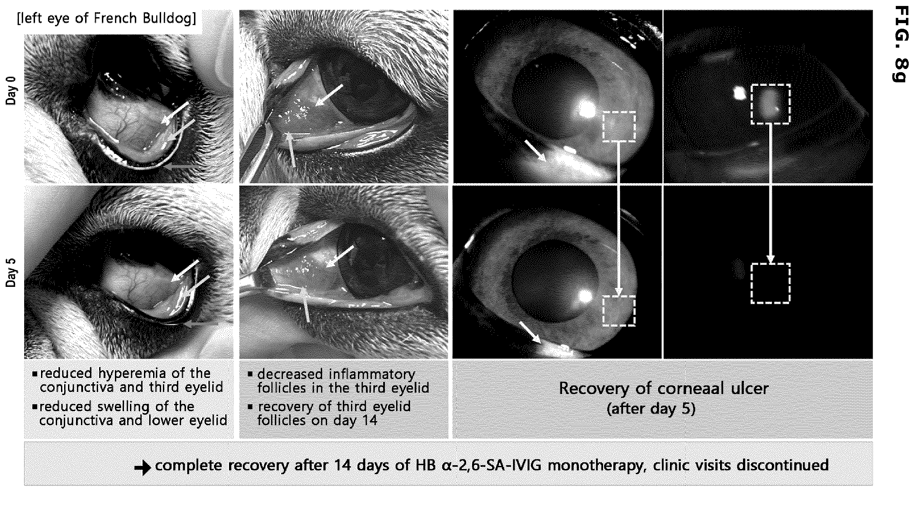

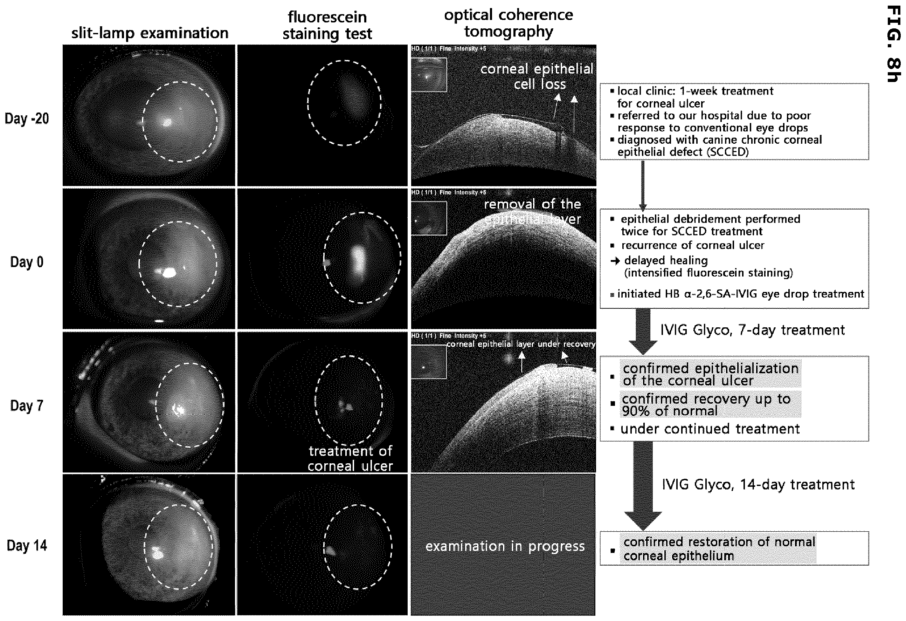

Based on the eight canine eye disease cases, a combination treatment was administered consisting of the conventional therapeutic agents at their guideline-recommended dosages along with HB α-2,6-SA-IVIG at a dose of 5 µg/100 µL per day (administered as 2.5 µg/50 µL twice daily, with PBS as the solvent). As a result, significant therapeutic effects were observed in all canine eye disease subjects (Table 4, FIGS. 8a-8h).

**[Table 4]**

| No | Diagnosis | Prescription (Secondary Veterinary Ophthalmic Hospital Treatment) | | Route of adminis tration | Duratio n of administ ration(d ay) | Therapeu tic Effect |
|---|---|---|---|---|---|---|
| 1 | Bilateral corneal epithelial defects and keratitis right eye with keratoconjunctivitis sicca | Conventional Treatment | -Optimmune bid | Instillat ion | 14 | None |
| | | | - An hypro | | | |
| | | Combinat ion Treatment | -Optimmune bid | Instillat ion | 16 | Improved |
| | | | - An hypro BID | | | |
| | | | - HB α-2,6-SA-IVIG | | | |
| 2 | Bilateral blepharitis, keratoconjunctivitis sicca | Conventi onal Treatment | - Maxitrol, | Instillat ion | 14 | No |
| | | | - optimmune, | | | |
| | | | - an-hypro | | | |
| | | Combinat ion Treatment | - Maxitrol, | Instillat ion | 16 | Recovere d |
| | | | - optimmune, | | | |
| | | | - an-hypro | | | |
| | | | - HB α-2,6-SA-IVIG | | | |
| 3 | Posterior rupture of the right eye(Day 0) enucleation followed by prosthetic eye implantation(Day 1) chronic keratitis in the right eye(Day 60) | Conventional Treatment | - Maxitrol | Instillat ion | 14 | None |
| | | | - optimmune | | | |
| | | Combinat ion Treatment | - Maxitrol | Instillat ion | 4 | Improved |
| | | | - optimmune | | | |
| | | | - HB α-2,6-SA-IVIG | | | |
| 4 | Malignant glaucoma in the right eye diagnosed, followed by enucleation and prosthetic eye implantation (Day 0) Corneal perforation and severe keratitis in the right eye (Day 86) | Conventi onal Treatment | - Prosthetic eye implantation | Instillat ion | 88 | None |
| | | | - Optimmune | | | |
| | | Combinat ion Treatment | - Corneal suturing | Instillat ion | 14 | Improved |
| | | | - Optimmune | | | |
| | | | - Levofloxacin | | | |
| | | | - HB α-2,6-SA-IVIG | | | |
| 5 | Bilateral pigmentary keratitis and severe chronic keratoconjunctivitis sicca | Conventi onal Treatment | - Optimmune, | Instillat ion | 14 | None |
| | | | - Maxitrol | | | |
| | | Combinat ion Treatment | - Optimmune | Instillat ion | 7 | Improved |
| | | | - Diquas | | | |
| | | | - HB α-2,6-SA-IVIG | | | |
| 6 | Bilateral qualitative dry eye | Conventi onal Treatment | - Fumelon | Instillat ion | 14 | None |
| | | | - An hypro | | | |
| | | Combinat ion Treatment | - Diquas | Instillat ion | 7 | Recovere d |
| | | | - HB α-2,6-SA-IVIG | | | |
| 7 | Keratohelcosis | Combinat ion Treatment | Chloramphenicol | Instillat ion | 14 | Recovere d |
| | | | - 0.3% hyaluronic acid | | | |
| | | | - HB α-2,6-SA-IVIG | | | |
| 8 | Recurrent chronic keratohelcosis | Conventi onal Treatment | - Ofloxacine | Instillat ion | 14 | None |
| | | | - an-hypro | | | |
| | | Combinat ion Treatment | - an-hypro | Instillat ion | 14 | Recovere d |
| | | | - HB α-2,6-SA-IVIG | | | |

Although specific embodiments of the present invention have been described in detail above, it will be apparent to those skilled in the art that such detailed descriptions are merely preferred examples and do not limit the scope of the invention. Accordingly, the substantial scope of the present invention shall be defined by the appended claims and their equivalents.

### Research Project Information

[Project Unique Number] 1345350798
[Project Number] 2016R1D1A1B01012721
[Funding Ministry] Ministry of Education, Korea
[Project Management Institution] National Research Foundation of Korea (NRF)
[Program Name] Basic Research Program (Individual Research, Ministry of Education)
[Project Title] Exploration of IVIG Alternatives Inducing DC-SIGN-Mediated Anti-inflammatory Effects
[Contribution Rate] 1/2
[Host Institution] Konkuk University
[Research Period] March 1, 2021 - February 28, 2022
[Project Unique Number] 1711191936
[Project Number] 2022R1A2C1009466
[Funding Ministry] Ministry of Science and ICT, Korea
[Project Management Institution] National Research Foundation of Korea (NRF)
[Program Name] Basic Research Program (Individual Research, MSIT)
[Project Title] Development of Anti-inflammatory Antibody Therapeutics Using ST6GAL1 Gene-Modified Myeloma Cell Lines
[Contribution Rate] 1/2
[Host Institution] Konkuk University
[Research Period] March 1, 2022 - February 28, 2025

## Claims

1. A pharmaceutical composition for preventing or treating dry eye syndrome or inflammatory eye disease, comprising sialylated immunoglobulin.

2. The pharmaceutical composition of claim 1,
wherein the immunoglobulin is isolated from intravenous immunoglobulin (IVIG).

3. The pharmaceutical composition of claim 1,
wherein the sialylated immunoglobulin is α-2,6-sialylated immunoglobulin.

4. The pharmaceutical composition of claim 1,
wherein the inflammatory eye disease is an inflammatory eye disease of the eye, oscular surface, posterior segment of the eye or periocular region.

5. The pharmaceutical composition of claim 4,
wherein the inflammatory eye disease is at least one selected from the group consisting of hordeolum (stye), blepharitis, lid wiper epitheliopathy, chalazion, eyelid infection, ulcerative and non-ulcerative keratitis, conjunctivitis, anterior uveitis, conjunctival ulcer, iridocyclitis, scleritis, episcleritis, optic neuritis, retinal vasculitis, chronic vasculitis, posterior uveitis, pan uveitis, meibomian gland dysfunction (MGD), and dacryoadenitis.

6. The pharmaceutical composition of claim 1,
wherein the pharmaceutical composition is co-administered with other therapeutic agent for treating dry eye syndrome or inflammatory eye disease.

7. The pharmaceutical composition of claim 6,
wherein the other therapeutic agent for treating dry eye syndrome or inflammatory eye disease is at least one selected from the group consisting of an anti-inflammatory agent, an immunosuppressant, an anti-glaucoma agent, artificial tears, a tear secretion stimulant and an antibiotic.

8. The pharmaceutical composition of claim 7,
wherein the pharmaceutical composition is in the form of an ophthalmic solution or an ointment.

9. The pharmaceutical composition of claim 3,
wherein the α-2,6-sialylated immunoglobulin, as compared to conventional intravenous immunoglobulin,
(i) enhances the expression of IL-10, an anti-inflammatory cytokine in ocular cells, by 50% or more; and/or
(ii) does not induce fibrotic side effects in the corneal stroma.

10. A ophthalmic composition, comprising α-2,6-sialylated immunoglobulin.

11. The ophthalmic composition of claim 10,
wherein the ophthalmic composition is in the form of a solution or an ointment.

12. The ophthalmic composition of claim 10,
wherein the ophthalmic composition is an artificial tear solution.

13. A method for treating dry eye syndrome or inflammatory eye disease in a non-human animal, comprising administering to the animal in need thereof the pharmaceutical composition of any one of claims 1 to 9.

14. The method of claim 13,
wherein the pharmaceutical composition is administered simultaneously, separately, or sequentially with other therapeutic agent for treating dry eye syndrome or inflammatory eye disease.
